# EUROPEAN PATENT APPLICATION

(11) **EP 3 865 173 A1**
(43) Date of publication of application: **18.08.2021**
(21) Application number: 20157468.8
(22) Date of filing: 14.02.2020
(51) Int. Cl.: A61M 31/00

(54) **NASAL DEVICE**

(71) Applicant: Beiter GmbH & Co. KG, 72517 Sigmaringendorf (DE)
(72) Inventor: PFÄFFLE, Rolf, 72517 Sigmaringendorf (DE)
(74) Representative: Kador & Partner PartG mbB

(57) **Abstract**

The present invention provides a nasal device for depositing at least one substance onto the *regio olfactoria.* The nasal device comprises a base structure having a distal section rigidly mechanically coupled to a proximal section. Furthermore, the nasal device comprises a lumen extending at least from the distal section to the proximal section of the base structure. The lumen comprises an opening at about an end region of the distal section for releasing the at least one substance. Additionally, the nasal device comprises a handling section being part of the proximal section and mechanically coupled thereto. Moreover, the nasal device comprises a protector, which forms a distal end of the nasal device for protection of a nasal inner region. Furthermore, the present invention provides a kit comprising the nasal device according to the present invention, optionally at least one substance and optionally an instruction for use.

## Description

### Field of the Invention

The present invention relates to a nasal device for depositing at least one substance onto the *regio olfactoria.* Furthermore, the invention relates to a kit comprising parts for building the nasal device.

### Background of the Invention

The blood-brain barrier represents an insurmountable barrier for many drugs used in the treatment of various diseases of the central nervous system, i.e. the brain, such as Alzheimer's disease, Parkinson's disease, multiple sclerosis and/or meningitis, and/or for developing agents for treatment of such diseases,. Therefore, ways to overcome the blood-brain barrier must be found to treat these diseases.

To date, one possibility has been to circumvent the blood-brain barrier by injecting directly into the cerebrospinal fluid (intrathecal) or directly into the brain ventricles (intraventricular). However, since the injection is invasive, this may cause discomfort in a treated subject and is associated with a very high risk of infection and injury while the implementation in a physically safe manner is difficult. Due to the difficult implementation, an application is to date restricted to medical centers and hospitals, i.e. an area-wide use is difficult to achieve, it is expensive and a repeated application is unpleasant for the treated subject.

Furthermore, the blood-brain barrier can hitherto be overcome by structurally modifying the active substance, such as lipophilization of the compound; utilization of transporter systems present in the endothelium of the blood-brain barrier; vectorization, for example, by using transgenic macrophages; or cationization of the compound etc. However, such modifications usually change the chemical properties and the therapeutic efficacy of the substance. Moreover, such modified compounds can subsequently cause considerable side effects such as organ damage due to their modification. In addition, the efficiency to cross the blood-brain-barrier by modification is normally restricted to small molecules with a molar mass of less than 500 g-mol⁻¹ to 600 g-mol⁻¹. Hence, this approach is therefore not applicable for many active ingredients.

Another possibility is to open the blood-brain barrier and thus making it basically continuous for molecules. However, this represents a massive intervention in the body's protective functions and is therefore associated with a considerable risk that toxic substances, which are normally retained by the blood-brain barrier, may enter into the brain.

Furthermore, it is well known that molecules without modification and size restriction can cross the blow-brain-barrier via the *regio olfactoria.* Neither an opening of the blood-brain barrier is required nor any invasive measure to administer the substance to the brain. However, at present, there is no device available allowing for the deposition of a substance onto the *regio olfactoria* in a physically safe manner for the subject, particularly without activating the immune system of the subject as outlined below, and allowing for an easy handling by a user ensuring an easy implementation of the deposition.

The *"regio olfactoria"* refers to an uppermost area of the nasal cavity and is a cell layer of the nasal mucosa covering an ethmoid bone. The term nasal cavity means the interior of the nose, which is coupled to the outer environment anteriorly through the nostrils of the subject, and posteriorly through the choanas to the nasopharynx of the subject. The ethmoid bone is a cranial bone traversed by fibers of an olfactory nerve, the *nervus olfactorius.*

The nasal inner region means the *regio olfactoria* and the ethmoid bone lying underneath the *regio olfactoria.*

A substance like a medicament can cross the blood-brain barrier by being deposited onto the *regio olfactoria.*

However, depositing of a substance onto the *regio olfactoria* is precarious. Puncturing or injuring of the nasal inner region and, particularly, the ethmoid bone, includes a risk to be lethal for the subject. Therefore, the device must ensure avoidance of puncturing or injuring of the nasal inner region and must ensure a non-invasive deposition of the substance, i.e. without puncturing body surfaces like mucosae.

Furthermore, contamination of adjacent mucosae, i.e. regions of the nasal mucosa adjacent to the *regio olfactoria* and/or the pharyngeal mucosa, by the substance need to be avoided. The immune system of the adjacent mucosae is highly active and contamination of the adjacent mucosae with a substance may trigger an immune response by a subject's body and may lead to a formation of antibodies and lymphocytes against the substance, e.g. an active ingredient. Such antibodies then may recognize the substance as a foreign substance and break it down. The antibodies and particularly the lymphocytes producing such antibodies, may be maintained in the subject's body even lifelong making any further and/or persistent administration of the substance to the subject impossible. Hence, triggering of an immune response must be prevented to ensure a persistent and repeated administration of the substance to the subject's brain.

### Description

In view of the above, there is a need for a device overcoming the above complications. Accordingly, it is an object of the present invention to provide a nasal device, which allows for depositing a substance onto the *regio olfactoria* in a physically safe manner for a subject, without harmful contamination of adjacent mucosae. Further, the device shall be easy in handling and safe to use for a user, such that the deposited at least one substance can cross the blood-brain-barrier and enter the brain without the requirement of being modified or restricted in size.

These and other objects, which become apparent upon reading the following description, are solved by the subject matter of the independent claims. The dependent claims refer to preferred embodiments of the invention.

The present invention is based on the concept of providing a nasal device for depositing at least one substance onto the *regio olfactoria* in a manner safe for the subject.

According to the invention, it is provided a nasal device for depositing at least one substance onto the *regio olfactoria.* The nasal device comprises a base structure having a distal section rigidly mechanically coupled to a proximal section. Furthermore, the nasal device comprises a lumen extending at least from the distal section to the proximal section of the base structure. The lumen comprises an opening at about an end region of the distal section for releasing the at least one substance. Additionally, the nasal device comprises a handling section being part of the proximal section and mechanically coupled thereto. Moreover, the nasal device comprises a protector, which forms a distal end of the nasal device for protection of a nasal inner region.

Furthermore, the present invention provides a Kit, comprising:
- the nasal device according to the present invention;
- optionally at least one substance; and
- optionally an instruction for use.

The nasal device is configured to deposit the at least one substance onto the *regio olfactoria* of a subject for a treatment of diseases of a central nervous system, such as Alzheimer's disease, Parkinson's disease, multiple sclerosis and/or meningitis, and/or for developing agents for treatment of such diseases.

The *"regio olfactoria"* means an uppermost area of a nasal cavity and is a cell layer of the nasal mucosa covering an ethmoid bone. The *regio olfactoria* may be the *regio olfactoria* of a subject. The subject may be an individual onto whose *regio olfactoria* the at least one substance is deposited. The subject may be a vertebrate, such as a mammal or human.

The nasal inner region means the *regio olfactoria* and the ethmoid bone lying underneath the *regio olfactoria.*

An general overview of the nasal device may be seen as having an elongated expansion with two opposite end sections designated "distal" and "proximal". Any parts or features of the nasal devices designated with "proximal" mean parts or features, which are located closer to the user's hand in normal use of the device, and correspondingly, any parts or features of the nasal device designated with "distal" mean parts or features, which are further away from the user's hand in normal use. For instance, the "proximal section of the base structure" of the nasal device means the section of the base structure, which is in normal use of the nasal device located closer to the user's hand and, correspondingly, the "distal section of the base structure" means the section of the base structure which is in normal use located further away from the user's hand. The "end region of the base structure" is a distal region of the base structure. Preferably, the end region has a length of 1.5 cm. The "distal end of the nasal device" designates the most distal protrusion of the nasal device. Furthermore, a "distal end of the base structure" as further described below, designates the most distal protrusion of the base structure. Furthermore, a "distal direction" or "distally directed" defines a direction that runs from proximal towards distal and, correspondingly, a "proximal direction" or "proximally directed" defines a direction that runs from distal towards proximal. An "axis proximal-distal" defines an axis running parallel to the distal direction and proximal direction, respectively.

The user may be typically the subject, a physician or a medical professional.

Preferably, the distal section of the base structure has an elongated expansion with dimensions fitting to be inserted into a nasal cavity. Thereby, the distal section can be inserted all the way up into the nasal cavity near the *regio olfactoria* of the subject.

Preferably, the distal section has a length of 2 cm - 20 cm, more preferably of 3 cm - 13 cm.

Preferably, the distal section is formed along its length providing a constant or varying outer diameter or a combination of both along the length. More preferably, the outer diameter refers to a maximum outer diameter of the cross-sectional shape of the distal section defined below and is essentially constant along the entire length of the distal section and/or declines from proximal to distal. Preferably, the distal section has a maximum outer diameter of 0.5 cm - 2 cm and more preferably of 0.7 cm - 1.5 cm. Most preferably, the maximum outer diameter is essentially constant along the entire length of the distal section to allow an easy and smooth insertion of the distal section into the nasal cavity.

Preferably, the cross-sectional shape of the distal section comprises only rounded and smooth convex structures and may be circular or of irregular shape like oval, eight-shaped, etc. More preferably, the cross-sectional shape is essentially constant along the entire length of the distal section. Thereby, any sharp and/or edgy structures are avoided which has the technical effect of minimizing the risk for injuring or puncturing the nasal inner region and allows for an easy and smooth insertion of the distal section into the nasal cavity.

Preferably, the distal section may comprise a curved section in a middle part of the distal section of the base structure. More preferably, the curvature of the curved section has an angle of 10° - 80°, most preferably 30° - 60°. Since most medical cutlery and equipment in otorhinolaryngology has a curved section, the nasal device having such curved section allows for a familiar and routine handling for the user. Due to the familiar and routine handling, the risk for injuring or puncturing the nasal inner region can be minimized further.

In the nasal device, the distal section of the base structure is rigidly mechanically coupled to the proximal section of the base structure.

The proximal section comprises the handling section. The handling section is mechanically coupled, preferably rigidly mechanically coupled, to the proximal section. Thereby, the handling section is via the proximal section rigidly mechanically coupled to the distal section of the base structure of the nasal device.

The term "rigidly mechanically coupling" as used herein means that in use, a movement of the proximal section of the nasal device is transmitted to the distal section of the nasal device without perceptible deformation of the nasal device. Hence, movements applied by the user to the proximal section, preferably to the handling region, of the nasal device are directly and immediately transmitted to the distal section, particularly to the opening of the lumen in the distal section of the nasal device and/or a source of electromagnetic radiation in the distal section of the nasal device as defined further below. Thereby the nasal device allows to deposit the at least one substance onto the *regio olfactoria* and/or to irradiate electromagnetic radiation for interaction with at least one substance already deposited onto the *regio olfactoria,* for instance, for curing at least one of the at least one substance as further described below.

The term "cure", "curing" or "curable" as used herein means harden, hardening or hardenable, i.e. it has to be interpreted in a physical sense.

The handling section may be any structure configured to be grasped by a user. Preferably, the handling section comprises a handle. The handling section allows a user to transfer its hand movements to the nasal device in order to control and direct movements of the distal section and/or to hold the nasal device stationary in place in a desired position.

Preferably, the base structure of the nasal device is entirely made of a rigid material and/or a combination of rigid materials. More preferably, the base structure is entirely made of a single rigid material as e.g. medical steel or plastic, most preferably, as one piece. This simplifies the manufacturing of the nasal device, avoids interfaces where dirt and germs can settle and facilitates cleaning of the device in case of a reuse. The rigid material may be any pharmaceutical and/or surgical acceptable material having rigidity such as a metal or plastic. The plastic is preferably a polymer such as polyethylene, polypropylene, polyvinylchloride, polystyrene, polyester, polyamide, polylactide acid, acrylonitrile styrene acrylate, acrylonitrile butadiene styrene, polyethylene terephthalate or polycarbonat or any combination thereof, optionally enforced with carbon fibers or other enforcement material. The metal is preferably a stainless steel, ferrous alloy, titan or tantalum or any combination thereof.

The protector forms the distal end of the nasal device for protection of the nasal inner region. Preferably, the protector exclusively forms the distal end of the nasal device.

The protector may be any modification, form, device, extension, measure etc. forming the distal end of the nasal device to reduce an impact to the nasal inner region, particularly to the ethmoid bone, in the event of a distally directed movement of the nasal device.

The protector might be a smooth rounding at a very distal tip of the nasal device and/or a configuration to absorb an impact to the nasal inner region in the event of a distally directed movement of the nasal device.

In case the protector has the smooth rounding, thereby any sharp and/or edgy harmful structures are avoided which minimizes the risk for injuring or puncturing the nasal inner region. More preferably, the protector has a smooth rounding with an edge radius of at least 0.5 mm or more, more preferably 1 mm or more.

In case the protector is configured to absorb an impact to the nasal inner region, the protector is configured to absorb an impact to a maximum pressure acceptable by the nasal inner region, particularly the ethmoid bone, without being punctured. The maximum pressure is preferably 10 N/cm² or less, more preferably of 5 N/cm² or less and most preferably 2 N/cm² or less. More preferably, the protector is configured to absorb the impact to the nasal inner region in the event of a distally directed movement along a distance of 0.5 cm - 0.2 cm, more preferably 1 cm - 0.2 cm, even more preferably 1.5 cm - 0.2 cm and most preferably 2 cm - 0.2 cm.

Preferably, the protector has a length of 2 cm - 0.2 cm, more preferably 1.5 cm - 0.2 cm, even more preferably 1 cm - 0.2 cm and most preferably 0.5 cm - 0.2 cm. More preferably, the protector absorbs the impact to the nasal inner region in the event of the distally directed movement over a distance which corresponds to the length of the protector or less, that is over a distance of 2 cm - 0.2 cm, more preferably 1.5 cm - 0.2 cm, even more preferably 1 cm - 0.2 cm and most preferably 0.5 cm - 0.2 cm.

Such configuration of the protector may be obtained by selection of: a material forming the protector; combinations of materials forming the protector; an elasticity of the material; a thickness or wall thickness of the material; a length of the material; a shape of the material; or any combination thereof.

Preferably, the protector comprises a plastically, elastically and/or telescopically deformable structure to absorb any impact to the nasal inner region.

The term "deformable" as used herein means bendable and/or compressible.

The term "plastically deformable" as used herein means a deformation, which is plastic.

The term "elastically deformable" as used herein means a deformation, which is elastic.

The term "telescopically" as used herein means telescopically deformable, thereby the protector is formed by several parts that can be inserted one into another to absorb the energy of the impact to the nasal inner region.

The term "flexible" as used herein is a generic term for deformable, plastically deformable and/or elastically deformable as defined above.

The bending, compressing, plastically deforming, elastically deforming and/or telescopically deforming requires a maximum pressure on the protector in the proximal direction which is at maximum a pressure acceptable the nasal inner region, particularly the ethmoid bone, without being punctured. The maximum pressure is preferably 10 N/cm² or less, more preferably of 5 N/cm² or less and most preferably 2 N/cm² or less. Thereby, the protector is configured to absorb the impact to the nasal inner region to a maximum pressure acceptable by the nasal inner region, particularly the ethmoid bone, without being punctured. The maximum pressure is preferably 10 N/cm² or less, more preferably of 5 N/cm² or less and most preferably 2 N/cm²

The protector has the technical effect that the nasal device allows for depositing the at least one substance to the *regio olfactoria* in a physically safe manner for the subject on whose *regio olfactoria* the substance is deposited. Potentially harmful and unwanted movements of the user, which might be uncoordinated, undirected and/or uncontrolled movements, are absorbed and/or distributed to a lager surface by the protector such that the nasal inner region is protected from being injured or punctured in case of being touched.

Furthermore, the protector even may allow that the nasal device can touch the nasal inner region without injuring or puncturing it. Since the distal section is rigidly mechanically coupled to the proximal section, the touching can be sensed by a user as a haptic feedback to indicate the user that the distal end of the nasal device is inserted all the way up into the nasal cavity and the *regio olfactoria* is reached. Thereby, the nasal device allows for an optimal deposition of the at least one substance as described further below and/or for an optimal interaction of the electromagnetic radiation with at least one of the deposited one or more substances as described further below.

The term "lumen" as used herein means an inner cavity of hollow structures, which can hold fluids or a fluid flow/stream without leakage.

Preferably, the lumen has a tubular shape with an elongated expansion.

Preferably, the lumen extends within the entire length of the distal section and in the proximal section of the base structure.

Preferably, the lumen extends within the distal section parallel to an axis along the elongated expansion of the distal section.

Preferably, the lumen has a length of 2 cm - 22 cm, more preferably 3 cm - 15 cm.

Preferably, the lumen has an inner diameter along its length suitable for a fluid comprising the at least one substance to flow/stream through it from the proximal section to the distal section thought the lumen and being released from the opening at the end region of the distal section. Preferably, the inner diameter is 0.5 mm - 6 mm, more preferably 1 mm - 3 mm. Preferably, the inner diameter of the lumen is essentially constant along the entire length of the lumen to avoid turbulences and to achieve a steady flow/stream of the fluid comprising the at least one substance.

Preferably, the lumen is a tube extending at least from the proximal section of the nasal device to the distal section of the nasal device.

Preferably, the tube extends within the entire length of the distal section and into the proximal section. A proximal opening of the tube might be fluidly coupled to a coupling link as described further below.

Preferably, the tube extends within the distal section parallel to the axis along the elongated expansion of the distal section.

Preferably, the tube has a length of 2 cm - 22 cm, more preferably 3 cm - 15 cm. In case a proximal end of the tube extends beyond the proximal section as described, further below, such additional length of such extension is not comprised in the said length of the tube.

Preferably, the tube has an inner diameter along its length suitable for the at least one substance to flow from the proximal section to the distal section thought the tube and being released from the opening at the end region of the distal section. Preferably, the inner diameter is 0.5 mm - 6 mm, more preferably 1 mm - 3 mm. Preferably, the inner diameter of the tube is essentially constant along the entire length of the tube to avoid turbulences in the flow of the at least one substance and to achieve a steady flow of the at least one substance.

Preferably, the nasal device comprises a support extending from the distal section to the proximal section, wherein the tube is releasable attached to the guidance.

Preferably, the releasable attachment of the tube to the support is accomplished in that the support is configured to accommodate the tube and that the tube can be easily inserted and/or removed from the guidance. The support may be configured for insertion and/or removal of the tube via a proximal opening of the guidance.

Preferably, the outer diameter of the tube is essentially constant along the entire length of the tube to allow for an easy insertion in and removal from the guidance. Preferably, the outer diameter of the tube is 0.2 cm - 1 cm and more preferably, 0.2 cm - 0.7 cm. Thereby, the tube can be removed from the base structure of nasal device by reversing the insertion movement. This results in the technical effect, that the base structure can be reused multiple times, whereas the tube, which may be in direct contact with the at least one substance during use of the nasal device, can be discarded and replaced by a new and/or clean tube.

Preferably, the support has an elongated, channel-like shape with an elongated expansion.

Preferably, the support extends within the entire length of the distal section and into the proximal section.

Preferably, the support extends within the distal section parallel to the axis along the elongated expansion of the distal section.

The channel-like shape may have sidewall(s), a distal opening and the proximal opening. The side wall(s) may be closed, partially open or open or any combination thereof along at least a part of the extension of the guidance.

The term "closed" in respect to the support means that the support forms a tubular structure without any holes or interruptions in the sidewall. The term "open" in respect to the support means that the support includes a continuous open slit parallel to the elongated expansion of the support, which extends along at least a part of the elongated expansion of the guidance. The width of the slit is smaller than the outer diameter of the tube in order to keep the tube attached to the guidance. Preferably, the support is open or is partially open with only a closed section forming a ring-like structure at the distal end of the base structure. The open or partially open construction has the technical effect, that the insertion and removal of the tube is facilitated, since the friction is minimized, adherence of the tube on the sidewall(s) of the support is decreased and possible impurities within the support can exit the support while inserting the tube. Furthermore, the tube is accessible and visible for instance for inspection or maintenance along the entire or at least partial length thereof. Moreover, in case the nasal device shall be re-used, the support can easily be cleaned and/or disinfected, since the inside of the support is accessible along the entire or at least partial length of the elongated expansion of the guidance. By the closed section forming a ring-like structure at the distal end of the base structure, any sharp and/or edgy structures are avoided, which has the technical effect of minimizing the risk for injuring or puncturing the nasal inner region and allows for an easy a smooth insertion of the distal section into the nasal cavity.

Preferably, the tube is made of any pharmaceutical and/or surgical acceptable material, which is flexible. More preferably, the tube is made of soft-PVC, silicone, polyethylene (PE), polyetheretherketone (PEEK), polycarbonate (PC), acrylnitril-butadien-styrol-copolymer (ABS) or a thermoplastic elastomere (TPE) or any combination thereof. Most preferably, the tube is a conventionally drainage and/or injection hose. Preferably, the tube has a wall thickness of 0.1 mm - 2 mm, more preferably 0.2 mm - 1.5 mm in order to provide the tube sufficient flexibility to be inserted and removed from the guidance.

Preferably, the protector is formed by a portion of the tube beyond a distal end of the base structure of the nasal device.

The portion of the tube beyond a distal end of the base structure of the nasal device is the portion of the tube, which distally protrudes from the distal end of the base structure and thereby forming the distal end of the nasal device. Preferably, the portion of the tube forming the protector exclusively forms the distal end of the nasal device.

In case, the portion of the tube might be smoothly rounded, the portion of the tube constitutes the protector having such rounding as described above and/or in case the portion of the tube might have a configuration to absorb the impact to the nasal inner region, the portion of the tube constitutes the protector which is configured to absorb an impact to the nasal inner region as described above.

In case the portion of the tube is configured to absorb an impact to the nasal inner region to the maximum pressure acceptable by the nasal inner region, particularly the ethmoid bone, without being punctured. The maximum pressure is preferably 10 N/cm² or less, more preferably of 5 N/cm² or less and most preferably 2 N/cm². More preferably, the portion of the tube is configured to absorb the impact to the nasal inner region in the event of a distally directed movement along a distance of 0.5 cm - 0.2 cm, more preferably 1 cm - 0.2 cm, even more preferably 1.5 cm - 0.2 cm and most preferably 2 cm - 0.2 cm.

The portion of the tube beyond the distal end of the base structure may have a length of 2 cm - 0.2 cm, more preferably 1.5 cm - 0.2 cm, even more preferably 1 cm - 0.2 cm and most preferably 0.5 cm - 0.2 cm, which correspond to the length of the protector as described above. More preferably, the portion of the tube absorbs the impact to the nasal inner region in the event of the distally directed movement over a distance which corresponds to the length of the protector or less, that is over a distance of 2 cm - 0.2 cm, more preferably 1.5 cm - 0.2 cm, even more preferably 1 cm - 0.2 cm and most preferably 0.5 cm - 0.2 cm.

Such configuration of the portion of the tube might be accomplished in that the entire tube is made of a material sufficiently flexible, the wall thickness of the portion of the tube may decrease in a distal direction and/or a material used for the portion of the tube is configured to be more flexible than the remaining tube. Thereby the portion of the tube is sufficiently elastically and/or plastically deformable to form the plastically and/or elastically structure of the protector to absorb the impact to the nasal inner region.

The opening at about the end region of the distal section is configured for releasing the at least one substance.

Preferably, the opening is configured to release the at least one substance in a direction distally away from the base structure onto the *regio olfactoria.*

For optimal spreading during releasing of the at least on substance onto the *regio olfactoria,* the opening should be positioned in a distance of 0.2 cm - 2 cm proximal to the *regio olfactoria.* The protector of the nasal device allows to optimally locating the opening in a distance of 0.2 cm - 2 cm to the *regio olfactoria.* As stated above, the protector may even allow that the *regio olfactoria* might be touched. In that the distal section rigidly mechanically coupled to the proximal section the touching can be sensed by a user as a haptic feedback to indicate to the user, that the distal end of the nasal device is inserted all the way up to the *regio olfactoria.* Thereby, the user is given a reference point in order to remove and/or reinsert the nasal device into the nasal cavity for positioning the opening in an optimal distance for disposal of 0.2 cm - 2 cm to the *regio olfactoria.*

In case the nasal device comprises a tube, which forms the lumen, the opening of the lumen at about the end region of the distal section is a distal opening of the tube.

Preferably, the nasal device comprises a source of electromagnetic radiation, which is arranged in the distal section, preferably about the end region of the distal section, and which is configured to interact with at least one of the at least one deposited substance.

The source of electromagnetic radiation may be any device that emits an electromagnetic radiation. For instance, the source of electromagnetic radiation may be an exit area of an optical fiber, as described further below, at about the end region of the distal section, which is configured to act as the source of electromagnetic radiation. Alternatively, the source of electromagnetic radiation may be a light-emitting diode (LED). The LED may be an LED of a generator of the electromagnetic radiation which is defined further below .

Preferably, the source of electromagnetic radiation is configured to emit the electromagnetic radiation in a direction extending distally away from the nasal device. The configuration of the emission is described further below.

The source of electromagnetic radiation may be configured to emit electromagnetic radiation as laser beam.

For optimal interaction of the electromagnetic radiation with an at least one substance deposited on the *regio olfactoria,* the source of electromagnetic radiation should be positioned in a distance of 0.2 cm - 2 cm proximal to the *regio olfactoria* during the interaction. The protector of the nasal device allows locating the source of electromagnetic radiation in a distance of 0.5 cm - 2 cm to the *regio olfactoria.* As stated above, the protector may even allow that the *regio olfactoria* might be touched. In that the distal section rigidly mechanically coupled to the proximal section, the touching can be sensed by a user as a haptic feedback to indicate to the user, that the distal end of the nasal device is inserted all the way up to the *regio olfactoria.* Thereby, the user is given a reference point in order to remove and/or reinsert the nasal device into the nasal cavity for positioning the source of electromagnetic radiation in a distance of 0.2 cm - 2 cm to the *regio olfactoria.*

The term "electromagnetic radiation" as used herein means any electromagnetic radiation comprising electromagnetic radiation in the non-visible range such as gamma radiation, X-radiation, ultraviolet (UV) radiation, infrared (IR) radiation, microwave radiation; or in the visible range; or any combination thereof. More preferably, the electromagnetic radiation comprises, preferably is, electromagnetic radiation in the non-visible range of electromagnetic radiation that means non-visible light. Even more preferably, the electromagnetic radiation is selected from the group consisting of X-radiation, UV-radiation, IR-radiation, microwaves or any combination thereof. Still more preferably, the electromagnetic radiation comprises, most preferably consists of ultraviolet radiation having a wavelength of 350 nm - 50 nm.

Preferably, the nasal device comprises a coupling link fluidly coupled to a proximal opening of the lumen, wherein the coupling link is configured to be fluidly coupled to a flow device for depositing a first substance; and/or wherein the coupling link is configured to be fluidly coupled to a reservoir device for depositing a second substance using an adapter device.

Preferably, the coupling link forms a hollow body defining a proximally open space.

The distal end of the space may be fluidly coupled to the proximal opening of the lumen for providing the at least one substance to the lumen.

In case the lumen is provided by the tube, the proximal opening of the lumen is the proximal opening of the tube.

The term "fluidly coupled" as used herein means that the coupling is sealing, that is a fluid exchange within the coupled parts can take place whereas the coupling is sealing at the interfaces between the coupled parts in that fluid cannot leak from the coupled parts and/or external fluids cannot enter from the exterior into the fluid. Furthermore, if not mentioned otherwise, the term "fluidly coupled" has to be interpreted in a broad sense in that the coupling is releasable, in that the coupled parts can be mounted and dismounted.

The fluid coupling may be accomplished by a proximally open space of the coupling link in combination with the flow device and/or the adapter device, which may be mutually sized and shaped, such that the flow device and/or the adapter device may be inserted into the space to establish the fluid coupling. Since the fluid coupling as defined above is releasable, the coupling link can be used for optionally mounting either the flow device or the reservoir device with the base structure in order to deposit the first and/or second substance

Additionally, the flow device and/or the adapter device may be releasable fixed in the space by a releasable mechanical fixation, for instance a bayonet fitting, screw coupling or press fit coupling. This results in the technical effect, that the flow device and/or the adapter device can easily be fluidly coupled, by mounting or dismounting to the coupling link and concomitantly providing a secured fluid coupling.

Preferably, the coupling link and/or the flow device and/or adapter device comprise a stop mechanism to restrict the insertion of the flow device and/or adapter device into the space, for instance the stop mechanism can be provided by the releasable mechanical fixation.

Preferably, the coupling link may be an integral part of the base structure of the nasal device arranged in the proximal section of the base structure. This results in the technical effect, that, even if the nasal device is already inserted into the nasal cavity of the subject, the coupling link is accessible to the user. Thereby, the coupling link can be used for optionally mounting and dismounting of for instance the flow device and/or the adapter device, while the base structure of the nasal device remains within the nasal cavity of the subject. This results in the technical effect, that a removal and reinsertion of the nasal device into nasal cavity of the subject is avoided, that is the nasal device remains in place. Furthermore, in that the coupling link is integral part of the base structure, the nasal device has very compact dimensions and the risk of the user of getting entangled is minimized. Therefore, potentially harmful and unwanted movements, for instance uncoordinated, undirected and/or uncontrolled movements, are not or are only minimally present, which minimizes the risk for injuring or puncturing the nasal inner region of the subject, particularly, the *regio olfactoria,* accelerates the treatment procedure for application of at least one substrate due to less operating steps and/or is more pleasant for the subject.

Alternatively, in case the lumen is provided by the tube, the coupling link may be provided in the nasal device as a non-integral part of the base structure. In this case, the proximal end of the tube extends beyond the proximal section of the nasal device and the proximal opening of the tube is fluidly coupled to the distal end of the coupling link. Thereby, the coupling link is due to the flexibility of the tube freely movable and without any fixed relation to the base structure. This results in the technical effect, that in case the nasal device is already inserted into the nasal cavity of the subject, transfer of movements of the coupling link for instance during mounting and dismounting it, with for instance the flow device, the reservoir device and/or adapter device to the base structure and therefore to the nasal inner region are minimized. Therefore, less potentially harmful and unwanted movements, for instance uncoordinated, undirected and/or uncontrolled movements, are not or are only minimally transmitted to the base structure and therefore to the nasal inner region, particularly, the *regio olfactoria,* while the nasal device remains in the nasal cavity of the subject. This results in the technical effect, that a removal and reinsertion of the nasal device into nasal cavity of the subject is avoided resulting in the already stated beneficial effects described above.

Most preferably, the coupling link is an integral part of the base structure of the nasal device for reasons of compactness of the nasal device.

### Flow device and flow generator

Preferably, the flow device includes a depot for providing the first substance.

The depot may hold the first substance. For instance, the first substance may be filled, for instance by pressing, into the depot. The depot preferably is filled with and holds the first substance prior to being fluidly coupled to the coupling link that is the depot is preloaded with the first substance.

The depot may be a cup-like structure, which has an aperture in the distal direction and a proximal bottom.

Preferably, the flow device is configured to provide the first substance to a fluid stream passing through the flow device in distal direction to entrain the first substance from the depot into the lumen.

The fluid stream may be a stream of any gaseous or liquid substance, for instance a gas, gas mixture, fume, fog, fume, solution, emulsion, suspension or any combination thereof. Preferably, the fluid stream is a gas. More preferably, the gas is air and/or carbon dioxide, most preferably, the gas is air as it is nontoxic and can be obtained from the environment.

For such configuration, the depot may be fixed by struts in a central position aligned to a proximal-distal axis of the flow device within a housing of the flow device. The struts may be essentially radially arranged on the outer surface of the depot to fix the depot to the housing of the flow device. The struts may be arranged in such a way that at least one flow opening between adjacent struts is provided in order to enable a fluid stream passing through the flow device in distal direction. On its proximal side, the depot may comprise a conical structure, wherein a base of the conical structure is arranged on the proximal side of the bottom of the depot and the tip of the conical structure faces proximal. Due to the conical structure, a fluid stream in a distal direction hitting the cone is deflected to flow more efficiently with less turbulences through the at least one flow opening of the flow device.

The at least one flow opening may be 1 - 40 openings, more preferably 2 - 20 and most preferably 4 - 8 flow openings. Thereby, large enough flow openings are provided for an efficient flow of the fluid stream, for avoidance of clotting of the flow openings, for easy cleaning of the flow device for reuse and/or for less difficult production of the flow device, while having strong enough struts to securely hold the depot in place.

The housing of the flow device and the proximal side of the depot of the flow device may form a hollow body defining a proximally open chamber. The conical structure further described above may be arranged within the chamber.

In case the flow device comprises a housing, the proximally open space of the coupling link interacts with the flow device via the outer surface of the housing of the flow device to establish the fluid coupling as described above.

Preferably, the coupling link and the flow device are configured to interact in such a way as to guide the fluid stream to enter the lumen via the coupling link. Such interaction can be provided when the flow device is fluidly coupled to the coupling link by inserting the flow device into the space of the coupling link.

Thereby, a cavity might be formed defined by the space of the coupling link and the flow device. The fluid stream passing through the flow device in distal direction enters the first cavity via the at least one flow opening and is swirled. The only exit of the cavity is the proximal opening of the lumen coupled to the distal end of the space. The aperture of the depot may be arranged in the cavity to give access to the swirled fluid stream to enter the aperture in order to entrain the first substance from the depot. The fluid stream, including the entrained first substance, can exit the cavity via the proximal opening of the lumen only, thereby, the fluid stream is guided to enter the lumen.

The flow device may comprise a stop mechanism to restrict the insertion of the flow device into the coupling link to ensure a formation of the cavity and enable the access of the fluid stream to the aperture.

Preferably, the nasal device comprises a flow generator for generating the fluid stream, wherein the flow generator is fluidly coupled to the flow device.

More preferably, the flow generator is configured to be fluidly coupled, wherein in this case the fluid coupling is not releasable e.g. via welding or bonding, or the fluid coupling is releasable as defined above, to the flow device. Most preferably, the flow generator is configured to be fluidly coupled, wherein the fluid coupling is releasable as defined above, to the flow device.

The fluid coupling, wherein the fluid coupling is releasable as defined above, may be accomplished to the proximally open chamber of the flow device by a distal connector of the flow generator, which may be mutually sized and shaped, such that the connector may be inserted into the open chamber to establish the fluid coupling.

Preferably, the flow device and the connector of the flow generator may be fluidly coupled by for instance a Luer/Lock-system, screw coupling, bayonet coupling, clip coupling, press fit coupling or any combination thereof. This results in the technical effect, that the flow generator can easily be mounted and dismounted from the flow device. Optionally, the flow device is already fluidly coupled to the flow generator before the flow device is fluidly coupled to the coupling link as described above.

The flow generator may comprise a flexible tubing line on which distal ending the connector of the flow generator is provided. The flexible tubing line has the technical effect that the flow generator is freely movable and without any fixed relation to the base structure. The flexible tubing line has the technical effect, that in case the nasal device is already inserted into the nasal cavity of the subject, transfer of movements of the flow generator, for instance during generation of the fluid flow such as manual pumping, to the base structure and therefore to the nasal inner region are minimized. Therefore, potentially harmful and unwanted movements, which might be uncoordinated, undirected and/or uncontrolled movements, are not or are only minimally transmitted to the base structure and therefore to the nasal inner region, particularly, the *regio olfactoria* while the nasal device remains in the nasal cavity of the subject. This results in the technical effect, that generation of the fluid flow can be conducted in a safe manner for the subject. The nasal device remains widely mechanically undisturbed at its place, which minimizes the risk for injuring or puncturing the *regio olfactoria* of the subject and/or is more pleasant for the subject.

Preferably, the flow generator is any device configured to generate the fluid stream. More preferably, the flow generator comprises a pump, for instance a manual pump or an electric pump; a gas cartridge; a blowing device, for instance a fan; or any combination thereof, most preferably a gas cartridge. More preferably, the pump is a manual pump such as hand pump, bellows, syringe, balloon pump, compressible balloon or an air pump.

Preferably, the coupling link and the flow generator are configured to interact in such a way as to guide the fluid stream to enter the lumen via the flow device and the coupling link.

Such interaction can be provided when the flow generator is fluidly coupled to the flow device, and the flow device is fluidly coupled to the coupling link as described above. The flow generator may comprise a connector and the flow generator is fluidly coupled to the flow device via the connector. Thereby, a second cavity is formed by the chamber of the flow device and the connector of the flow generator. The conical structure of the flow device is arranged within the second cavity, wherein the tip of the conical structure is positioned such that the distally directed fluid stream generated by the flow generator and entering the second cavity via the connector hits the proximal tip of the conical structure. Thereby, the fluid stream in a distal direction entering the second cavity is deflected by the conical structure to flow more efficiently with less turbulences through the at least one flow opening. Then, the fluid stream passing through the flow device in distal direction enters the first cavity via the at least one flow opening and is swirled as described above.

Preferably, the flow generator is configured to control a release of the first substance from the opening of the lumen of the nasal device. More preferably, the flow generator is configured to control the deposition of the first substance onto the *regio olfactoria* by controlling the release of the first substance from the opening of the lumen the nasal device.

For such configuration the flow generator may be configured for a user to control the flow generation of the fluid stream, thereby the entrainment of the first substance from the depot by the fluid stream and/or the release of the first substance from the opening of the lumen of the nasal device and hence the deposition of the first substance are controlled. For instance, the duration, intensity, frequency of interruptions and interval length, etc. of the flow generation might be controlled. The flow generator may comprise a manual pump, wherein the flow of the fluid stream is then controlled manually. Alternatively, the flow generator may comprise an electrical pump, wherein the flow generation is then controlled electrically, for instance, by a switch.

### Adapter device and reservoir device

The adapter device may provide a passage opening along the axis distal-proximal to fluidly couple the reservoir device with the lumen.

The reservoir device may be configured to be fluidly coupled to the adapter device.

More preferably, the reservoir device is configured to be fluidly coupled, wherein in this case the fluid coupling is not releasable e.g. via welding or bonding, or the fluid coupling is releasable as defined above, to the adapter device. Most preferably, the reservoir device is configured to be fluidly coupled, wherein the fluid coupling is releasable as defined above, to the adapter device.

The fluid coupling, wherein the fluid coupling is releasable as defined above, may be accomplished by a proximal connection of the adapter device in combination with a distal connection of the reservoir device, which may be mutually sized and shaped in order to establish the fluid coupling.

Preferably, the connection of the adapter device and the connection of the reservoir device may be fluidly coupled by for instance by a Luer/Lock-system, screw coupling, bayonet coupling, clip coupling, press fit coupling, any combination thereof. Such fluid coupling has the technical effect that the reservoir device can be easily mounted and dismounted from the adapter device and the adapter device may be cleaned and reused and/or coupled with a further reservoir device. Optionally, the reservoir device is already fluidly coupled to the adapter before the adapter device is fluidly coupled to the coupling link as described above.

The reservoir device may comprise a flexible tubing line having a distal end fluidly coupled to the connection of the reservoir device is provided. The flexible tubing line has the technical effect that the reservoir device is freely movable in respect to the base structure. The flexible tubing line has the technical effect, that in case the nasal device is already inserted into the nasal cavity of the subject, transmittance of movements of the reservoir device, for instance during generation of a flow of the at least one substance such as by manual pumping, to the base structure and therefore to the nasal inner region are minimized. Therefore, potentially harmful and unwanted movements, which might be uncoordinated, undirected and/or uncontrolled movements, are not or are only minimally transmitted to the base structure and therefore to the nasal inner region, particularly, the *regio olfactoria* while the nasal device remains in the nasal cavity of the subject. This results in the technical effect, that generation of the fluid flow can be conducted in a safe manner for the subject. The nasal device remains widely mechanically undisturbed at its place, which minimizes the risk for injuring or puncturing the *regio olfactoria* of the subject and/or is more pleasant for the subject.

Preferably, the reservoir device is any device for maintaining and releasing a second substance and for generating a flow of the second substance. More preferably, the reservoir device comprises a pump, for instance a manual pump or an electric pump; a gas cartridge; a blowing device, for instance a fan; or any combination thereof. More preferably, the pump is a manual pump such as hand pump, bellows, syringe, balloon pump, compressible balloon or an air pump and most preferably a syringe.

Preferably, the reservoir device is configured to control the release of the second substance onto the *regio olfactoria* from the opening of the lumen the nasal device.

For such configuration the reservoir device is configured for a user to control the release of a flow of the second substance, thereby the release of the second substance from the opening of the lumen of the nasal device is controlled. For instance, the duration, intensity, frequency of interruptions and interval length, etc. of the flow are controlled. The reservoir device may comprise a manual pump, wherein the flow of the at least one substance is controlled manually. Alternatively, the reservoir device may comprise an electrical pump, wherein the flow of the at least one substance is controlled electrically, for instance by a switch. Preferably, the reservoir device is a manual pump like a conventional syringe. The reservoir device contains prior to being operated the second substance, that is, the reservoir device is preloaded with the second substance. While being operated, a flow of the second substance is released from the opening of the lumen of the nasal device for depositing the second substance onto the *regio olfactoria.*

The at least one substance may be the first substance and/or the second substance.

The term "substance" as used herein refers to a single compound or a composition of different compounds and/or ingredients, e.g. active ingredients.

The first substance may be provided in the form of particles. The particles may be dry powder, microparticles, vesicles or nanoparticles or any combination thereof. Particles can be easily filled, for instance by pressing, into the depot of the flow device and hold by the flow device. Furthermore, particles can be efficiently entrained from the depot by the fluid stream passing though the flow device in distal direction into the lumen, to deposit the first substance on the *regio olfactoria.*

Preferably, the first substance comprises a medicament for the treatment of diseases of the central nervous system such as Alzheimer's, Parkinson's disease, epilepsy, amyotrophic lateral sclerosis, meningitis or multiple sclerosis.

Preferably, the size of the particles first substance is 0.01 µm - 500 µm, more preferably 0.1 µm - 200 µm, even more preferably 1 µm - 100 µm and most preferably 5 µm - 10 µm. Thereby, the depot of the flow device can optimally hold the first substance filled into the depot and additionally the first substance can efficiently entrained from the depot by the fluid stream passing though the flow device in the distal direction into the lumen, to deposit the first substance on the *regio olfactoria.*

The second substance may be provided in the form of a gel. Preferably, the gel is curable by electromagnetic radiation and/or self-curing. More preferably, the gel is a polymer composition curable by electromagnetic radiation and/or self-curing. Even more preferably, the gel is curable, preferably, solely curable by electromagnetic radiation. Most preferably, the gel is not self-curing.

Preferably, the second substance is a pharmaceutical acceptable substance. The second substance may comprise a medicament for the treatment of diseases of the central nervous system such as Alzheimer's, Parkinson's disease, epilepsy, amyotrophic lateral sclerosis, meningitis or multiple sclerosis.

Preferably, after curing, the cured second substance is suitable to adhere to the *regio olfactoria.* More preferably, the adherence of the cured second substance lasts 1 - 6 weeks, preferably at least 2 - 5 weeks and most preferably 3 - 4 weeks. After this time, the cured second substance preferably dissolves, decomposes and/or peels off from the *regio olfactoria* or clears the *regio olfactoria* in any other way.

Therefore, preferably, the nasal device might be configured to be used in a first step for depositing the first substance onto the *regio olfactoria* and in a second step for depositing and curing the second substance onto the *regio olfactoria.* Thereby, a combination of the first substance and the second substance can be deposited.

In case the first substance is in the form of particles comprising the medicament for the treatment of diseases of the central nervous system and the second substance is in the form of the gel which is curable by electromagnetic radiation, a persistent and repeated administration of the first substance, that is the medicament, to the *regio olfactoria* to be provided to a brain can be ensured at a correct site without contaminating adjacent mucosae. Hence, by using the nasal device for depositing, activating of the immune response against the medicament is avoided. Thereby a persistent and repeated administration of the medicament becomes possible, since the immune response against the medicament is avoided.

Furthermore, the configuration of the nasal device for depositing in a first step the first substance, in case the first substance is a medicament and in the form of particles has the technical effect, that the first substance in the form of particles is released from the opening of the lumen of the nasal device as a dust. The fluid stream entrains the particles and thereby forms the dust. Such dust easily distributes within the nasal inner region and the particles can be very easily deposited on the *regio olfactoria,* however, also adjacent mucosae may be contaminated. However, the contamination is only very transient, since the adhesion of the deposited first substance is transient, preferably less than 15 min, more preferably less than 5 min and most preferably less than 1 min. Thereby, the first substance, which is accidentally deposited to regions other than the *regio olfactoria,* for instance adjacent mucosae, and therefore contaminates these mucosae is quickly removed by the body functions of the subject and activation of the immune response against the medicament is avoided. The contamination may be too short as to activate the immune response against the medicament. Hence, since the nasal device is configured for depositing the first substance in the first step, in case the first substance is a medicament and in the form of particles, a durable contamination of these mucosae is avoided. Furthermore, a faulty deposition may be corrected. Moreover, an activation of the immune system against the first substance, particularly the medicament, is prevented, because the interaction with the mucosae is too short to activate the immune response.

In contrast to this, a persistent administration of the first substance comprising the medicament to the *regio olfactoria* is clearly desired. Only a persistent application to the *regio olfactoria* allows the medicament to cross the blood-brain-barrier, enter the brain and develop its beneficial effect. Hence, the medicament as correctly deposited to the *regio olfactoria* must be prevented from being quickly removed therefrom by the body functions of the subject.

Consequently, the nasal device is also configured for depositing in a second step the second substance, in case the second substance is provided in the form of a gel curable by electromagnetic radiation, in order to ensure a persistent administration of the medicament to the brain. As stated above, the medicament must be prevented from being removed from the *regio olfactoria* or at least a portion of the *regio olfactoria* to enter the brain and to develop its beneficial effect. This is achieved in that the nasal device is configured for depositing the gel curable by electromagnetic radiation on top of the first substance onto the *regio olfactoria* or a portion of the *regio olfactoria.*

In case the nasal device comprises a source of electromagnetic radiation in the distal section configured to interact with the at least one substance which is the second substance in the form of a gel curable by electromagnetic radiation, the second substance is cured by emitting the electromagnetic radiation to the second substance after depositing the second substance onto the *regio olfactoria.* Such a cured second substance forms a kind of patch by which the medicament is persistently adhered to the *regio olfactoria.* Thereby, the nasal device allows providing the medicament to enter a brain and allows a persistent administration of the medicament to the subject. Preferably, the persistent administration of the medicament lasts as long as the medicament is still present on the deposition area. Furthermore, since the adherence of the cured second substance, that is the patch, may last only for a certain time as stated above the persistent administration of the medicament may at maximum last as long as the adherence takes place. After this time, the *regio olfactoria* is cleared for repeating the persistent administration. As stated above, the nasal device has the advantage that it can be reused, particularly the base structure can be reused multiple times, and hence, the nasal device allows for such repeated administration.

Due to allowing the use of a second substance curable by electromagnetic radiation, the nasal device has the advantage, that depositing and curing of the second substance are separate active steps. In case the second substance is accidentally deposited to regions other than the *regio olfactoria,* for instance the nasal mucosa and pharyngeal mucosa and these mucosae are already contaminated with the first substance, the second substance, which is not yet cured, may be removed e.g. by body functions of the subject. Hence, the first substance is not durable adhered to these mucosae by depositing accidentally the second substance on top. Furthermore, the device allows specifically curing only portions of the deposited second substance by electromagnetic radiation, which have been deposited correctly to the *regio olfactoria,* whereas portions accidentally deposited to adjacent mucosae are not subjected to curing. Thereby, the device allows correcting a faulty deposition of the second substance on unwanted regions in that the curing step is not performed by not activating the source of electromagnetic radiation. The nasal device subsequently allows repeating the deposition of the second substance until it has been correctly deposited on the *regio olfactoria.*

Consequently, the nasal devices allows that a durable contamination of adjacent regions of the nasal mucosa with the medicament is avoided. Furthermore, the nasal devices allows that a faulty deposition by the user of the first and second substance on such adjacent regions can be corrected. Moreover, the nasal devices allows that an activation of the immune system against the first substance, particularly the medicament, is prevented.

The device also allows to use a second substance that might be self-curing as mentioned above. This has the advantage that an additional operational step may be avoided, since an active curing does not need to be performed. However, in case the second substance is accidentally deposited on regions other than the *regio olfactoria* and these mucosae are already contaminated with the first substance, the medicament will be durable adhered to these mucosae by depositing the second substance on top. The medicament may not be removed quickly by the body functions of the subject. A durable contamination of the mucosae is given in this case. Moreover, a faulty deposition of the first and second substance by the user on such adjacent regions may not be corrected and an activation of the immune system against the first substance, particularly the medicament, may be expected. Therefore, the second substance may be provided in the form of a gel, which is solely curable by electromagnetic radiation as described above.

The nasal device may be configured for depositing and curing the at least one substance, wherein the at least one substance is a combination-substance, which is provided in form of a gel and curable by electromagnetic radiation and comprising the medicament as described above.

Preferably, the source of electromagnetic radiation interacts with one of the at least one deposited substance to cure the one substance.

Preferably, the at least one substance is curable by electromagnetic radiation, most preferably the second substance.

Preferably, the source of the electromagnetic radiation is configured to be controlled by a user in respect to an intensity of the electromagnetic radiation emitted by the source of electromagnetic radiation.

For such configuration, the source of electromagnetic radiation may be operatively coupled to a control unit operated by a user, like a switch, for instance an electrical switch controlling the electric supply to a generator of the electromagnetic radiation as described below, or a mechanical light shade. Thereby, the intensity of the electromagnetic radiation can be controlled via the duration of the emittance of the electromagnetic radiation; the frequency of interruptions and interval lengths of the emittance of the electromagnetic radiation; and/or the energy of the electromagnetic radiation, for instance via dimming or shading the electromagnetic radiation.

Preferably, the nasal device comprises an optical fiber extending from the distal section to the proximal section, wherein an exit area of the optical fiber at about the end region of the distal section is configured to act as the source of electromagnetic radiation.

Preferably, the optical fiber has an elongated expansion.

The optical fiber may have a shape and dimensions to accommodate in the distal section of the base structure.

Preferably, the optical fiber extends within the distal section parallel to the axis along the elongated expansion of the distal section.

Preferably, the optical fiber extends from the distal section to the proximal section of the base structure. More preferably, the optical fiber extends within the entire length of the distal section and into the proximal section.

Preferably, the optical fiber has a length of 2 cm - 22 cm, more preferably 3 cm - 15 cm.

Preferably, the optical fiber is any fiber conventionally used to guide electromagnetic radiation and known to the person skilled in the art.

Preferably, the optical fiber as a diameter of 0.005 mm to 4 mm.

Preferably, the nasal device comprises an optical coupler, which is optically coupled to a proximal end of the optical fiber.

Preferably, the nasal device comprises a generator of the electromagnetic radiation optically coupled to the optical coupler.

The generator of the electromagnetic radiation is any device suitable to generate the electromagnetic radiation as defined above. For instance, the generator of the electromagnetic radiation may comprise a light-emitting diode (LED) and power supply like a battery.

Most preferably, the source of the electromagnetic radiation is configured to be controlled by a user in respect to an intensity of the emitted electromagnetic radiation by controlling the generation of the electromagnetic radiation via an energy supply to the generator of the electromagnetic radiation.

For this configuration the generator of the electromagnetic radiation comprises the control unit as defied above, which is an electrical switch, thereby, the generation of the electromagnetic radiation by the generator of the electromagnetic radiation is controlled via the electrical switch.

The optical coupler is any device suitable to operatively connect the optical fiber with the generator of the electromagnetic radiation. Preferably, the connection has to be interpreted in a broad sense in that the connection is releasable, in that the connected parts can be mounted and dismounted. This has the advantage, that the generator of the electromagnetic radiation can be replaced, e.g. in case it gets broken or needs to be recharged, while the remaining nasal device can be equipped with a replacement generator of the electromagnetic radiation and is then ready for immediate further use.

Preferably, the generator of the electromagnetic radiation may be arranged in the proximal section of the base structure and optically coupled to the optical fiber via the optical coupler.

The generator of the electromagnetic radiation may be releasable and/or permanently attached, preferably releasable attached, to the proximal section.

The base structure of the nasal device may comprise a mounting at the proximal section to releasable attach the generator of the electromagnetic radiation. Thereby, the generator of the electromagnetic radiation may be operatively coupled to the optical fiber via the optical coupler. The mounting has the advantage, that the generator of the electromagnetic radiation can be replaced, e.g. in case it gets broken or needs to be recharged, while the remaining nasal device can be equipped with a replacement generator of the electromagnetic radiation and is then ready for immediate further use. The attachment of the generator of the electromagnetic radiation to the base structure provides the advantage that the nasal device has compact dimensions and the risk of a user of being entangled is minimized. Therefore, potentially harmful and unwanted movements, which may be uncoordinated, undirected and/or uncontrolled movements, are not or are only minimally transmitted to the base structure and therefore to the nasal inner region, particularly, the *regio olfactoria* may be minimized, while the nasal device is still inserted into the nasal cavity of the subject.

Alternatively, the generator of the electromagnetic radiation may comprise a flexible coupling element to operatively link the electromagnetic radiation of the generator with the optical coupler, for instance a tubular guide for electromagnetic radiation extending beyond the proximal end of the base structure of the nasal device. This results in the technical effect that the generator of the electromagnetic radiation is freely movable and without any fixed relation to the base structure. The flexible coupling element has the technical effect that in case the nasal device is still inserted into the nasal cavity of the subject, transmittance of movements of the generator of the electromagnetic radiation, instance during activation of the source of electromagnetic radiation by the user controlling the generator, to the base structure and therefore to the nasal inner region are minimized. Therefore, potentially harmful and unwanted movements of the user, which might be uncoordinated, undirected and/or uncontrolled movements, are not or are only minimally transmitted to the base structure and therefore to the nasal inner region, particularly, the *regio olfactoria* while the nasal device remains in the nasal cavity of the subject. This results in the technical effect, that generation and controlling of the electromagnetic radiation may be conducted in a safe manner for the subject. The nasal device remains widely mechanically undisturbed at its place, which minimizes the risk for injuring or puncturing the *regio olfactoria* of the subject and/or is more pleasant for the subject.

Preferably:
the source of the electromagnetic radiation is configured to emit electromagnetic radiation in a solid angle extending distally away from the distal section; and
the solid angle of the emitted electromagnetic radiation overlaps with an area of the deposited at least one substance at the *regio olfactoria* if using the nasal device for curing it.

Preferably, the at least one substance is curable by electromagnetic radiation, most preferably the second substance.

The solid angle of the light source defines the area irradiated by the electromagnetic radiation.

Most preferably, the source of electromagnetic radiation is configured to emit electromagnetic radiation as laser beam; and the laser beam overlaps with the deposited at least one substance at the *regio olfactoria* using the nasal device in order to cure it.

Preferably, the nasal device is for use in combination with a conventional endoscope. The endoscope can be equipped with an imaging device and/or surgical tool, preferably an imaging device. Thereby, the correct positioning of the nasal device within the nasal cavity and/or the distance of the distal end of the nasal device to the *regio olfactoria* can be controlled and monitored.

An imaging device is for instance an endoscopic camera or any other device capable of producing a visible image.

A surgical tool is for instance a knife, scissors, pliers, tweezers or needle or a combination thereof.

In one embodiment, the nasal device comprises an imaging device and/or surgical tool in the distal section.

In another embodiment, the nasal device does not comprise an imaging device and/or surgical tool in the distal section.

Furthermore, the present invention provides a kit, comprising:
- the nasal device according to the present invention;
- optionally at least one substance; and
- optionally an instruction for use.

All features of the kit are defined as described above.

### Figures

In the following, the present invention will be illustrated by examples and by referring to the following figures.
- **Fig. 1a**: sketches in a lateral view the nasal device with a tube deposited in the support, wherein the protector is formed by a distal portion of the tube.
- **Fig. 1b**: sketches in an isometric view in proximal direction the nasal device of **Fig. 1a****.**
- **Fig. 2a**: sketches in a lateral view a base structure of a nasal device with attached protector.
- **Fig. 2b**: sketches in an isometric view in proximal direction of the base structure with attached protector of **Fig. 2a****.**
- **Fig. 3**: sketches in an isometric view in proximal direction a flow device.
- **Fig. 4a**: sketches in a lateral, cross-sectional view a nasal device with a fluidly coupled flow device and flow generator and a generator of the electromagnetic radiation attached in a support.
- **Fig. 4b**: sketches an enlarged detail of the **Fig. 4a** with a fluidly coupled flow device.
- **Fig. 5**: sketches in a lateral view, cross-sectional a nasal device with a fluidly coupled adapter device and reservoir device and a generator of the electromagnetic radiation attached in a mounting.

### Embodiments of the invention

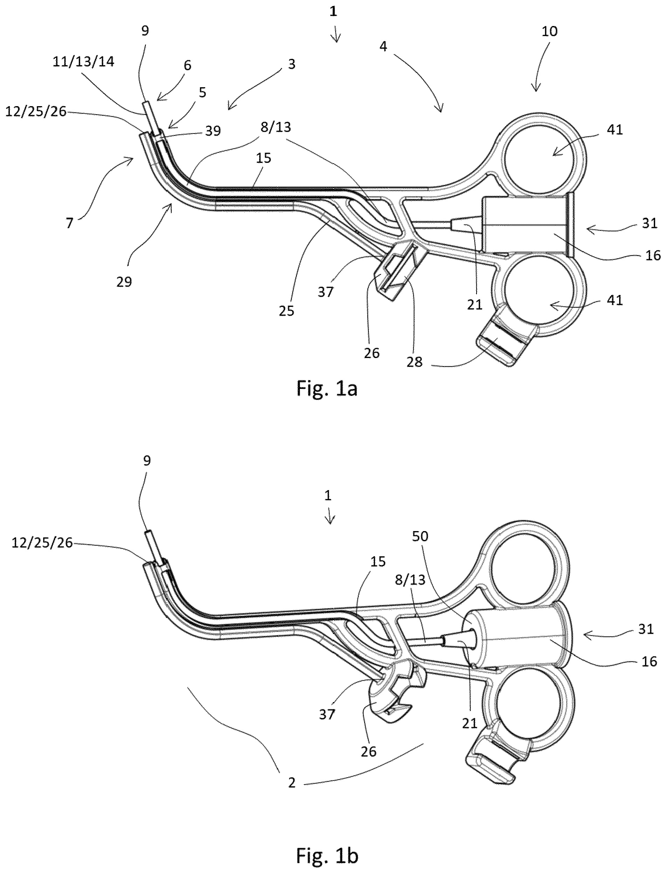

**Fig. 1a** sketches in a lateral view and **Fig. 1b** sketches in an isometric view in proximal direction the nasal device (1) with a base structure (2) for depositing at least one substance onto the *regio olfactoria.* The nasal device comprises a base structure (2) having a distal section (3) rigidly mechanically coupled to a proximal section (4). The distal section (3) has an elongated expansion with an axis along the elongated expansion.

Furthermore, the nasal device comprises a lumen (8) extending from the distal section (3) to the proximal section (4) of the base structure (2). Thereby, the lumen (8) extends within the distal section (3) parallel to the axis along the elongated expansion of the distal section (3). The lumen (8) is provided by a tube (13) releasable attached by insertion into a support (15), wherein the support (15) and the tube (13) also have an elongated expansion extending along the distal section (3) and along the entire length of the distal section (3) and to the proximal section (4).

The support (15) is partially open, which means that the support (15) includes a continuous open slit (38) parallel to the elongated expansion of the support (15). The width of the slit (38) is smaller than the outer diameter of the tube in order to keep the tube attached to the guidance. The support (15) is hence partially open with a closed section forming a ring-like structure (39) at the distal end (5) of the base structure (3).

Furthermore, the nasal device (1) comprises an opening (9) provided by the opening of the tube (13) at about an end region (7) of the distal section (3) for releasing the at least one substance.

Furthermore, the lumen (8) has a proximal opening (21) which is provided by a proximal opening (21) of the tube (13). The proximal opening (21) of the tube is fluidly coupled to a distal end of a coupling link (16). The coupling link (16) forms a hollow body defining a proximally open space (31) and the distal end of the coupling link (16) is a distal bottom (50) of the coupling link (16) with a bore (51) for fluidly coupling the lumen (8).

Furthermore, the nasal device (1) comprises a protector (11) forming a distal end (6) of the nasal device (1) for protection of a nasal inner region. The protector (11) is formed by a portion (14) of the tube (13) beyond a distal end (5) of the base structure (3) of the nasal device (1). Thereby, the portion (14) of the tube (13) distally protrudes from the distal end (5) of the base structure (2) and thereby forming the distal end (6) of the nasal device (1). In other words, the distal end (6) of the nasal device (1) is exclusively formed by the portion (14) of the tube (13), thereby forming the most distal protrusion of the nasal device (1) serving as the protector (11).

Additionally, the nasal device (1) comprises a handling section (10) including a handle (41), wherein the handling section (10) is part of the proximal section (4) and rigidly mechanically coupled thereto.

Moreover, the distal section (3) comprises a curved section (29) in a middle part of the distal section (3) of the base structure (2).

Moreover, the nasal device (1) comprises a source of electromagnetic radiation (12) at the distal section (3). Precisely, the source (12) of electromagnetic radiation is arranged at the end region (7) of the distal section (3). The source (12) of electromagnetic radiation is provided by an exit area (26) of an optical fiber (25).

The optical fiber (25) extends from the distal section (3) to the proximal section (4) of the base structure (2). Thereby, the optical fiber (25) has an elongated expansion, which extends within the distal section (3) parallel to the axis along the elongated expansion of the distal section (3) and essentially parallel to the elongated expansion of the support (15) and the lumen (8) in the proximal section (4) of the base structure (2).

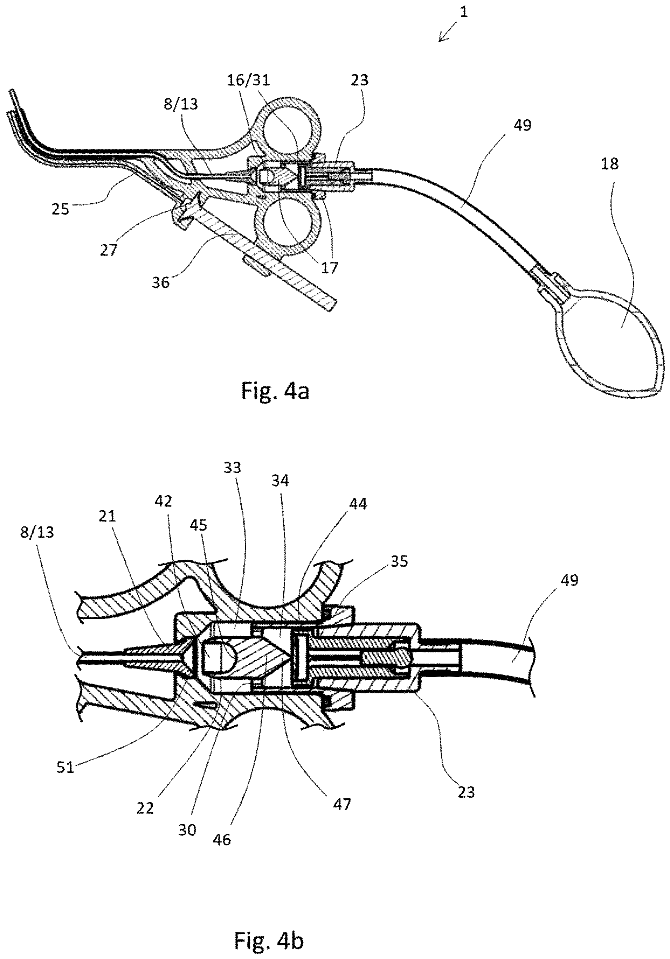

Additionally, the nasal device (1) comprises an optical coupler (27) coupled to a proximal end (37) of the optical fiber (25) in the distal section (3) of the base structure (2) of the nasal device (1). Furthermore, the base structure (2) comprises at the proximal section (4) a mounting (28) for a releasable attachment of a generator (36) of the electromagnetic radiation for operatively coupling the generator (36) of the electromagnetic radiation to the optical fiber (25) via the optical coupler (27). The nasal device (1) with attached generator (36) of the electromagnetic radiation is sketched in **Figs. 4a** and **4b****.**

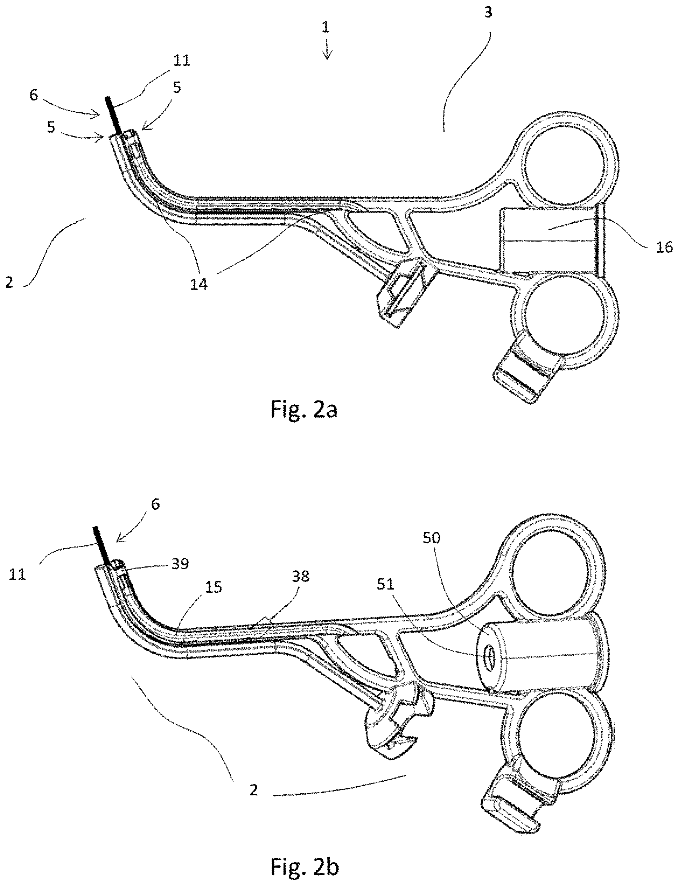

**Fig. 2a** sketches a lateral view and **Fig. 2b** sketches an isometric view in proximal direction the base structure (2) of a nasal device (1) with a protector (11) attached at the distal end (5) of the base structure (2). The support (15) is shown without an attached tube (13). The protector (11), which is in this case not formed by a portion (14) of the tube (13), is arranged at the distal end (5) of the base structure (2), thereby forming the distal end (6) of the nasal device (1). In other words, the distal end (6) of the nasal device (1) is exclusively formed by the protector (11), thereby, the protector (11) forms the most distal protrusion of the nasal device (1).

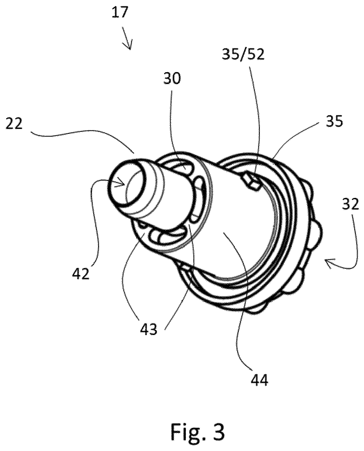

**Fig. 3** sketches in an isometric view in proximal direction a flow device (17) and **Fig. 4a** and **Fig. 4b** sketch in a lateral, cross-sectional view the flow device (17) fluidly connected to the coupling link (16). The flow device (17) includes a depot (22) for providing the first substance (substance is no shown). The depot (22) has a cup-like structure with an aperture (42) in the distal direction and a proximal bottom (45). The depot (22) fixed by struts (43) in a central position aligned to a proximal-distal axis of the flow device (17) within a housing (44) of the flow device (17). The struts (43) are essentially radially arranged on the outer surface of the depot (22) and fix the depot (22) with the housing (44) of the flow device (17). The struts (43) are arranged to provide spaces (30) between adjacent struts (43) in order to provide at least one flow opening (30) in a distal direction to allow a fluid stream passing through the flow device (17) in distal direction. On its proximal side the depot (22) comprises a conical structure (46), wherein a base of the conical structure (46) is arranged on the proximal side of the bottom (45) of the depot (22) and a tip (47) of the conical structure (46) faces proximal. The housing (44) and the proximal side of the depot (22) of the flow device may form a hollow body defining a proximally open chamber (32). The conical structure (46) is arranged within the chamber (32).

Furthermore, **Fig. 4a** and **Fig. 4b** sketch in a lateral, cross-sectional view the flow device (17) and a flow generator (18) fluidly coupled to the coupling link (16) of the nasal device (1). The flow device (17) is inserted into the space (31) of the coupling link (16), thereby forming a first cavity (33) defined by the space (31) of the coupling link and the flow device (17). A fluid stream passing through the flow device via the at least one flow opening (30) in distal direction enters the first cavity (33) and is swirled. The only exit of the first cavity (33) is the proximal opening (21) of the lumen (8) provided by the tube (13) which is coupled to the distal end of the space (31), that is the distal end of the coupling link (16). The aperture (42) of the depot (22) is arranged in the first cavity (33) to give access to the swirled fluid stream to enter the aperture (33) in order to entrain the first substance from the depot (22). The flow device (17) comprises a stop mechanism (35) to restrict the insertion of the flow device (17) into the coupling link (16) to ensure the formation of the first cavity (33) and the access of the fluid stream to the aperture (42). Furthermore, the flow device (17) comprises a releasable mechanical fixation (52) to releasable fix the flow device (17) within the space (32).

Furthermore, in the nasal device (1) of **Fig. 4a** and **Fig. 4b** a flow generator (18) is fluidly coupled to the flow device (17). Hence, distally the flow device (17) is fluidly coupled to the coupling link (16) and proximally the flow device (17) is fluidly coupled to the flow generator (18).

The flow generator (18) comprises a connector (23) and the flow generator (18) is coupled to the flow device (17) via the connector (23). Thereby, a second cavity (34) is formed by the chamber (32) of the flow device (17) and the connector (23) of the flow generator (18). The conical structure (46) of the flow device (17) is arranged within the second cavity (34), wherein the tip (47) of the conical structure (46) is positioned such that a distally directed fluid stream generated by the flow generator (18) and entering the second cavity (34) via the connector (23) hits the proximal tip (47) of the conical structure (46) and is deflected by the conical structure (46) to flow more efficiently with less turbulences through the at least one flow opening (30) into the first cavity (33) as described above.

The flow generator (18) comprises a flexible tubing line (49) on which distal ending the connector (23) of the flow generator (18) is provided. The depicted flow generator (18) is a balloon pump.

Furthermore, in **Fig. 4a** the generator (36) of electromagnetic radiation is attached in the mounting (28) as described above for **Fig. 1a**. Thereby, the generator (36) of electromagnetic radiation is operatively coupled to the optical fiber (25) via the optical coupler (27).

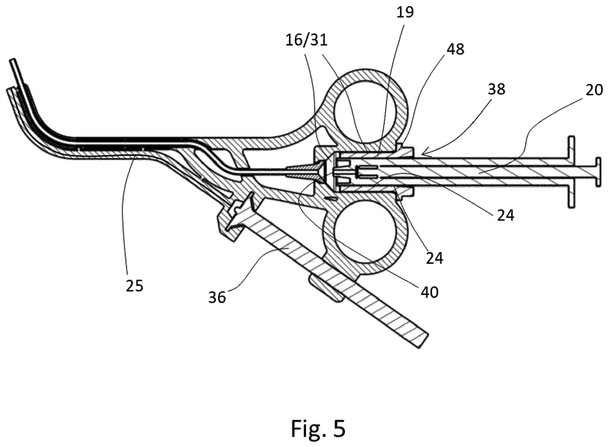

The **Fig.** 5 sketches in a lateral, cross-sectional view the nasal device of **Fig. 4a****,** however, instead of the flow device (17) and the flow generator (18) an adapter device (19) and a reservoir device (20) are fluidly coupled to the coupling link (16).

The adapter device (19) is inserted into the space (31) of the coupling link (16) to establish the fluid coupling. The reservoir device (20) is fluidly coupled to the adapter device (19), wherein in the depicted embodiment the reservoir device (20) is a conventional medical syringe inserted into the adapter device (19). The adapter device (19) may provide a passage opening (40) along the axis distal-proximal to fluidly couple the reservoir device (20) with the lumen (8). Hence, distally the adapter device (19) is fluidly coupled to the coupling link (16) and proximally the adapter device (19) is fluidly coupled to the flow generator reservoir (20).

The adapter device (19) comprises a stop mechanism (48) to restrict the insertion of the adapter device (19) into the coupling link (16).

### Listing of reference signs

- 1: nasal device
- 2: base structure
- 3: distal section of the base structure (2)
- 4: proximal section of the base structure (2)
- 5: distal end of the base structure (2)
- 6: distal end of the nasal device (1)
- 7: end region of the distal section (3)
- 8: lumen
- 9: opening
- 10: handling section
- 11: protector
- 12: source of electromagnetic radiation (12)
- 13: tube
- 14: portion of the tube (13) beyond a distal end (5)
- 15: guidance
- 16: coupling link
- 17: flow device
- 18: flow generator
- 19: adapter device
- 20: reservoir device
- 21: proximal opening of the lumen (8)/(13)
- 22: depot
- 23: connector of the flow generator (18)
- 24: connection of the reservoir device (20)
- 25: optical fiber
- 26: exit area
- 27: optical coupler
- 28: mounting
- 29: curved section
- 30: flow opening
- 31: space of the coupling link (16)
- 32: chamber of the flow device (17)
- 33: first cavity formed by space (30) and flow device (17)
- 34: second cavity formed by chamber (31) and connector (23)
- 35: stop mechanism of the flow device (17)
- 36: generator of electromagnetic radiation
- 37: proximal end of the optical fiber
- 38: slit of the support (15)
- 39: closed section forming a ring-like structure
- 40: passage opening
- 41: handle
- 42: aperture of the depot (22)
- 43: struts
- 44: housing of the flow device (17)
- 45: proximal bottom of the depot (22)
- 46: conical structure
- 47: tip of the conical structure (46)
- 48: stop mechanism of the adapter device (20)
- 49: flexible tubing line
- 50: distal bottom
- 51: bore
- 52: releasable mechanical fixation

## Claims

1. Nasal device (1) for depositing at least one substance onto the *regio olfactoria,* comprising:
- a base structure (2) having a distal section (3) rigidly mechanically coupled to a proximal section (4);
- a lumen (8) extending at least from the distal section (3) to the proximal section (4) of the base structure (2), wherein the lumen (8) comprises an opening (9) at about an end region (7) of the distal section (3) for releasing the at least one substance;
- a handling section (10) being part of the proximal section (4) and mechanically coupled thereto; and
- a protector (11) forming a distal end (6) of the nasal device (1) for protection of a nasal inner region.

2. Nasal device (1) according to claim 1, comprising:
- a source (12) of electromagnetic radiation in the distal section (3) configured to interact with at least one of the at least one deposited substance.

3. Nasal device (1) according to claim 1 or 2, wherein the protector (11) is configured to absorb an impact to the nasal inner region, particularly to the *regio olfactoria,* in the event of a distally directed movement of the nasal device (1).

4. Nasal device (1) according to any of the preceding claims, wherein the lumen (8) is a tube (13) extending at least from the proximal section (4) of the nasal device (1) to the distal section (3) of the nasal device (1), and wherein the protector (11) is formed by a portion (14) of the tube (13) beyond a distal end (5) of the base structure (2) of the nasal device (1).

5. Nasal device (1) according to any of the preceding claims, comprising a support (15) extending from the distal section (3) to the proximal section (4), and wherein the tube (13) is attached to the support (15).

6. Nasal device (1) according to any of the preceding claims, comprising a coupling link (16) fluidly coupled to a proximal end (21) of the lumen (8), the coupling link (16) is configured to be fluidly coupled to a flow device (17) for depositing a first substance; and/or
the coupling link (16) is configured to be fluidly coupled to a reservoir device (20) for depositing a second substance using an adapter device (19).

7. Nasal device (1) according to any of the preceding claims, wherein the flow device (17) includes a depot (22) for providing the first substance.

8. Nasal device (1) according to any of the preceding claims, wherein the flow device (17) is configured to provide the first substance to a fluid stream passing through the flow device (17) in distal direction to entrain the first substance from the depot (22) into the lumen (8).

9. Nasal device (1) according to any of the preceding claims, wherein the coupling link (16) is configured to be fluidly coupled to the flow device (17) and/or the adapter device (19).

10. Nasal device (1) according to any of the preceding claims, comprising a flow generator (18) for generating the fluid stream, wherein the flow generator (18) is fluidly coupled to the flow device (17).

11. Nasal device (1) according to any of the preceding claims, wherein the first substance is in the form of particles.

12. Nasal device (1) according to any of the preceding claims, wherein the second substance is in the form of a gel.

13. Nasal device (1) according to any of the preceding claims, wherein the second substance is curable by electromagnetic radiation.

14. Nasal device (1) according to any of the preceding claims, comprising an optical fiber (25) extending from the distal section (3) to the proximal section (4), wherein an exit area (26) (20) of the optical fiber (25) at about the end region (7) of the distal section (3) is configured to act as the source (12) of electromagnetic radiation.

15. Nasal device (1) according to any of the preceding claims, comprising an optical coupler (27), which is optically coupled to a proximal end (37) of the optical fiber (25) for coupling with a generator (36) of the electromagnetic radiation.

16. Nasal device (1) according to any of the preceding claims, wherein:
- the source of electromagnetic radiation is configured to emit electromagnetic radiation in a solid angle extending distally away from the distal section (3); and
- the solid angle of the emitted electromagnetic radiation overlaps with an area of a deposited at least one substance at the *regio olfactoria* if using the nasal device (1) for curing it.

17. Kit, comprising:
- the nasal device (1) according to any of the preceding claims;
- optionally at least one substance; and
- optionally an instruction for use.
